(19) Europäisches Patentamt
European Patent Office
Office européen des brevets

(11) EP 0 708 101 B1

(12) # FASCICULE DE BREVET EUROPEEN

(45) Date de publication et mention de la délivrance du brevet:
**09.12.1998 Bulletin 1998/50**

(51) Int Cl.[6]: **C07D 401/06**, A61K 31/415, A61K 31/445, C07D 471/04, C07D 401/14

(21) Numéro de dépôt: **95402330.5**

(22) Date de dépôt: **19.10.1995**

(54) **Dérivés de pipéridine utiles comme antagonistes des récepteurs des neurokinines**

Piperidinderivate, verwendbar als Neurokinin-Rezeptor-Antagonisten

Piperidine derivatives, useful as neurokinin receptor antagonists

(84) Etats contractants désignés:
**AT BE CH DE DK ES FR GB GR IE IT LI LU NL PT SE**

(30) Priorité: **21.10.1994 FR 9412580**

(43) Date de publication de la demande:
**24.04.1996 Bulletin 1996/17**

(73) Titulaire: **ADIR ET COMPAGNIE**
**92415 Courbevoie Cédex (FR)**

(72) Inventeurs:
- **de Nanteuil, Guillaume**
  **F-92150 Suresnes (FR)**
- **Remond, Georges**
  **F-78000 Versailles (FR)**
- **Portevin, Bernard**
  **F-78990 Elancourt (FR)**
- **Bonnet, Jacqueline**
  **F-75013 Paris (FR)**
- **Canet, Emmanuel**
  **F-75005 Paris (FR)**
- **Birrell, Graham**
  **Edimburgh EH10 4JU, Scotland (GB)**

(56) Documents cités:
**EP-A- 0 396 282**
**EP-A- 0 512 901**
**EP-A- 0 515 240**
**WO-A-94/10146**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 708 101 B1

## Description

La présente invention concerne de nouveaux dérivés de pipéridine, leur procédé de préparation et les compositions pharmaceutiques qui les contiennent, qui possèdent des propriétés antagonistes sélectives et puissantes vis-à-vis des récepteurs des neurokinines.

Les neurokinines forment une famille de neuropeptides possédant à la partie C terminale une analogie de structure : Phe-X-Gly-Leu-Met. Ces neuropeptides, substance P (SP), neurokinine A (NKA) et neurokinine B (NKB) induisent une contraction rapide des fibres musculaires lisses par opposition aux contractions lentes développées par la bradykinine. Largement représentées au niveau du corps humain, en particulier au niveau du système nerveux central et du système nerveux périphérique, leurs effets agonistes endogènes s'effectuent via des récepteurs spécifiques avec une affinité préférentielle pour $NK_1$, $NK_2$, $NK_3$ respectivement pour SP, NKA et NKB. Ils sont impliqués dans de nombreux processus physiologiques ou physiopathologiques tels que nociception, vasoperméabilité, contractions des fibres musculaires lisses, hypersécrétions et immuno-modulations (OTSUKA M. et coll., Physiol. Rev. 73, 229-308, 1993).

Les propriétés antagonistes des composés de l'invention vis-à-vis des récepteurs des neurokinines et plus particulièrement des récepteurs $NK_1$, les rendent utilisables notamment dans le traitement de la douleur, des processus inflammatoires d'origines diverses, des troubles gastro-intestinaux, de l'asthme, des allergies, des troubles urologiques, de la migraine et des maladies du système nerveux central.

Les composés les plus proches de l'Art Antérieur sont plus particulièrement décrits dans les brevets EP 396282, US 4791121, US 4791120, EP 515901 ou WO 94/10146.

La présente invention concerne plus particulièrement les composés de formule (I):

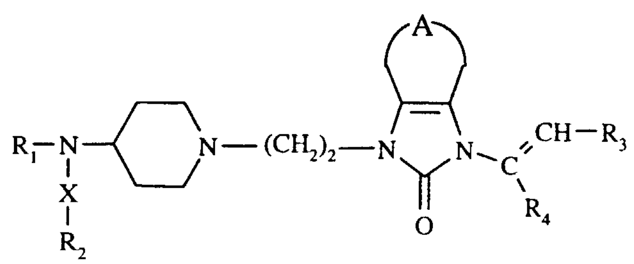

(I)

dans laquelle:

- $R_1$ représente un groupement alkyle ($C_1$-$C_6$) linéaire ou ramifié, phényle, naphtyle, pyridyle ou thiényle, chaque groupement phényle, naphtyle, pyridyle ou thiényle étant éventuellement substitué par un ou plusieurs atomes d'halogène ou groupements hydroxy, alkoxy ($C_1$-$C_6$) linéaire ou ramifié, alkyle ($C_1$-$C_6$) linéaire ou ramifié, trihalogénométhyle ou 1-hydroxy-2,2,2-trifluoroéthyle,

- $R_2$ représente un atome d'hydrogène, un groupement alkyle ($C_1$-$C_6$) linéaire ou ramifié (substitué ou non par un ou plusieurs groupements alkoxy ($C_1$-$C_6$) linéaire ou ramifié ou phényle, amino ou phtalimido), phényle (substitué ou non par un ou plusieurs atomes d'halogène ou groupements alkyle ($C_1$-$C_6$) linéaire ou ramifié, alkoxy ($C_1$-$C_6$) linéaire ou ramifié, hydroxy, trihalogénométhyle), cycloalkyle ($C_3$-$C_7$), pipéridino ou amino (substitué ou non par un ou deux groupements alkyle ($C_1$-$C_6$) linéaire ou ramifié),

- X représente un groupement CO ou $SO_2$,

- $R_3$ représente un atome d'hydrogène ou un groupement alkyle ($C_1$-$C_6$) linéaire ou ramifié,

- $R_4$ représente un groupement alkyle ($C_1$-$C_6$) linéaire ou ramifié, un groupement phényle (substitué ou non par un ou plusieurs atomes d'halogène ou groupements alkyle ($C_1$-$C_6$) linéaire ou ramifié, alkoxy ($C_1$-$C_6$) linéaire ou ramifié, hydroxy ou trihalogénométhyle) ou un groupement trihalogénométhyle,

ou bien, $R_3$ et $R_4$forment ensemble avec les atomes de carbone qui les portent un groupement cycloalkényl ($C_3$-$C_7$),

- A représente, avec les atomes de carbone auxquels il est attaché, un cycle phényle, naphtyle, pyridyle, chaque cycle phényle, naphtyle, pyridyle étant éventuellement substitué par un ou plusieurs atomes d'halogène, ou groupements alkyle ($C_1$-$C_6$) linéaire ou ramifié, alkoxy ($C_1$-$C_6$) linéaire ou ramifié, hydroxy, amino, nitro ou trihalogénométhyle,

leurs isomères, les sels d'ammonium quaternaire de la pipéridine correspondants ainsi que leurs sels d'addition à un acide pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, oxalique, méthane sulfonique, camphorique, etc...

Les sels d'ammonium quaternaire de la pipéridine peuvent être réalisés par exemple à l'aide d'iodure de méthyle.

L'invention s'étend également au procédé de préparation des composés de formule (I) caractérisé en ce que l'on utilise comme produit de départ :

**soit** une amine de formule (II) :

$$R_1\text{-}NH_2 \qquad (II)$$

dans laquelle $R_1$ a la même signification que dans la formule (I),
que l'on fait réagir sur une pipéridone de formule (III) :

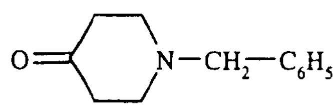

(III)

pour conduire au composé de formule (IV),

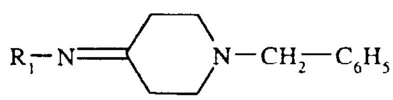

(IV)

dans laquelle $R_1$ a la même signification que dans la formule (I),
qui subit une réduction en présence d'un hydrure métallique,
pour conduire au composé de formule (V) :

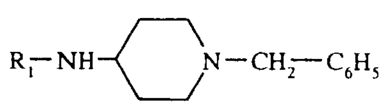

(V)

dans laquelle $R_1$ a la même signification que dans la formule (I),
que l'on met en réaction avec un anhydride, un chlorure d'acide ou le phosgène (suivie d'une réaction avec une amine secondaire),
pour conduire au composé de formule (VI) :

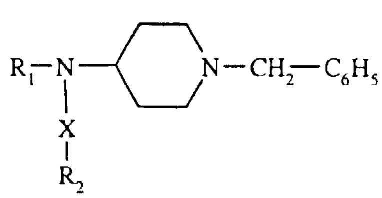

(VI)

dans laquelle $R_1$, $R_2$ et X ont la même signification que dans la formule (I),
qui subit une débenzylation par hydrogénation catalytique, transfert d'hydrogène (en présence de formiate d'ammonium) ou par déalkylation (en présence d'α-chloroformiate de chloréthyle),
pour conduire au composé de formule (VII) :

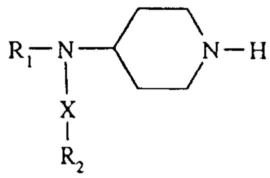

(VII)

dans laquelle $R_1$, $R_2$ et X ont la même signification que dans la formule (I), que l'on fait réagir avec un composé de formule (VIII) :

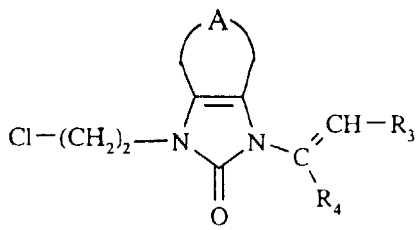

(VIII)

dans laquelle A, $R_3$ et $R_4$ ont la même signification que dans la formule (I), pour conduire au composé de formule (I),
**soit** une pipéridine de formule (IX) :

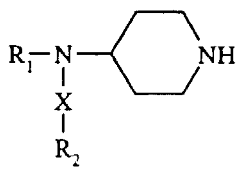

(IX)

dans laquelle $R_1$, $R_2$ et X ont la même signification que dans la formule (I), que l'on fait réagir avec l'époxyéthane, pour conduire au composé de formule (X) :

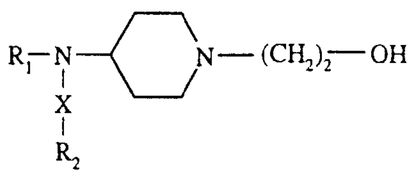

(X)

dans laquelle $R_1$, $R_2$ et X ont la même signification que dans la formule (I), qui subit l'action du chlorure de thionyle pour conduire au composé de formule (XI) :

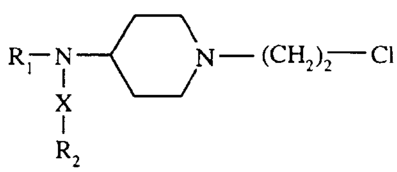

(XI)

dans laquelle $R_1$, $R_2$ et X ont la même signification que dans la formule (I), que l'on fait réagir avec le composé de formule (XII) :

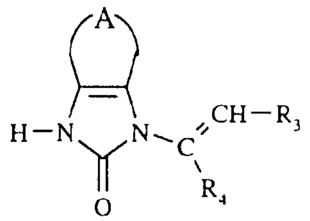

(XII)

dans laquelle A, $R_3$ et $R_4$ sont tels que définis dans la formule (I), pour conduire au composé de formule (I), composé de formule (I) :

- qui peut être, le cas échéant, purifié selon une technique classique de purification,
- dont on sépare, le cas échéant, les isomères selon une technique classique de sépararation,
- que l'on transforme, si on le souhaite, en ses sels d'addition à un acide pharmaceutiquement acceptable ou en sel d'ammonium quaternaire de la pipéridine.

Les composés de l'invention possèdent des propriétés pharmacologiques très intéressantes. Ce sont des ligands spécifiques des récepteurs des neurokinines qui possèdent en particulier des propriétés antagonistes particulièrement intenses vis-à-vis des récepteurs $NK_1$. Les récepteurs $NK_1$ seraient plus particulièrement impliqués dans la régulation de la transmission de la douleur, de l'oedème induit par augmentation de la vasoperméabilité, des phénomènes sécrétoires au niveau trachéo-bronchique et gastro-intestinal, de la salivation, du contrôle ventilatoire et du tonus vasculaire, et de l'activation des cellules participant aux processus inflammatoires.

La présente invention a également pour objet les compositions pharmaceutiques renfermant comme principe actif au moins un composé de formule (I) seul ou en combinaison avec un ou plusieurs excipients ou véhicules inertes non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 100 mg pour un traitement en 1 à 3 prises par 24 heures.

Les exemples suivants illustrent l'invention et ne la limitent en aucune façon.

Les produits de départ utilisés sont des produits connus ou préparés selon des modes opératoires connus.

Les composés décrits dans les préparations A à M sont des intermédiaires de synthèse utiles dans la préparation des composés de formule (I).

Les structures chimiques des composés décrits dans les exemples ont été déterminées selon les techniques spectroscopiques habituelles (résonance magnétique du Proton et du carbone 13, spectre de masse...).

***PREPARATION A*** ***: 1-(2-Chloroéthyl)-3-isopropényl-2(3H)-benzimidazolone***

A 50 mmoles de 1-isopropényl-2(3H)benzimidazolone dans 150 ml de diméthylformamide sont ajoutées 200 mmoles de 1-bromo-2-chloroéthane et 55 mmoles de carbonate de potassium. L'ensemble est maintenu 48 heures sous agitation. Il est alors versé dans de l'eau glacée et extrait au dichlorométhane. Les phases organiques sont lavées à l'eau, séchées et évaporées. Le résidu est alors repris par de l'éther. La phase éthérée est lavée par de la potasse 2N, puis par de l'eau, séchée et évaporée et conduit au produit attendu.

***PREPARATION B*** ***: 3-Isopropényl-2(3H)-benzimidazolone***

A 500 mmoles de 2-aminoaniline dans 150 ml de xylène chauffées à 120°C sont ajoutés 1 ml d'une solution de potasse à 47 % puis 530 mmoles d'acétoacétate de méthyle en solution dans 20 ml de xylène. L'ensemble est chauffé et le mélange eau/méthanol formé est éliminé au moyen d'un appareil Dean-Stark. L'ensemble est alors porté 3 heures à reflux. Après refroidissement à 40°C, on ajoute 83 ml de potasse à 47 % et 55 ml d'eau. La phase aqueuse alcaline est neutralisée par de l'acide acétique. Le produit attendu cristallise alors ; il est filtré, lavé à l'eau et séché.

*Point de fusion : 120° C*

***PREPARATION C* : 3-Cyclopent-1-ènyl-2(3H)-benzimidazolone**

100 mmoles de 2-aminoaniline et 135 mmoles de 2-éthoxycarbonylcyclopentanone dans 50 ml de xylène sont portées 5 heures à reflux en éliminant à l'aide d'un appareil Dean-Stark l'eau et l'éthanol formés. Après refroidissement, le produit attendu cristallise, il est alors filtré, lavé au xylène puis à l'hexane puis séché.

*Point de fusion : 158-160° C*

***PREPARATION D* : 3-Isopropényl-2(3H)-naphto[2,3-d]imidazolone**

Le produit attendu est obtenu selon le procédé décrit dans la préparation C à partir de 2,3-diaminonaphtalène et d'acétoacétate d'éthyle.

*Point de fusion : 200-203° C*

***PREPARATION E* : 3-Isopropényl-5,6-dichloro-2(3H)-benzimidazolone**

A 150 mmoles de 2-amino-4,5-dichloroaniline dans 50 ml de xylène agitées à 120°C sont ajoutés successivement 0,3 ml de potasse à 47 % et 160 mmoles d'acétoacétate de méthyle dans 20 ml de xylène. L'ensemble est porté à reflux 4 heures en éliminant le mélange eau/méthanol formé au moyen d'un appareil Dean-Stark. Après addition de 26 ml de potasse à 47 % et de 17 ml d'eau, la phase aqueuse est lavée au xylène puis neutralisée par de l'acide acétique. Le produit attendu cristallise, est filtré, lavé et séché.

*Point de fusion : 190-195° C*

***PREPARATION F* : 3-Isopropényl-7-méthyl-2(3H)-benzimidazolone**

Le produit attendu est obtenu selon le procédé décrit dans la préparation C à partir de 2-amino-3-méthylaniline et d'acétoacétate d'éthyle.

*Point de fusion : 195° C*

***PREPARATION G* : 3-Isopropényl-4-nitro-2(3H)-benzimidazolone**

A 100 mmoles de 2-amino-3-nitro aniline dans 50 ml de xylène portées à 120°C, sont ajoutés 0,3 ml de potasse à 47 % puis 115 mmoles d'acétoacétate de méthyle. L'ensemble est porté 4 heures à reflux et le mélange eau/méthanol formé est éliminé au moyen d'un appareil Dean-Stark. 18 ml de potasse à 47 % et 200 ml d'eau sont alors ajoutés. La phase aqueuse est lavée par du xylène, amenée à pH = 6 par de l'acide chlorhydrique 12N puis extraite à l'acétate d'éthyle et le produit attendu est purifié par chromatographie sur colonne de silice en utilisant comme éluant un mélange dichlorométhane/méthanol (95/5).

***PREPARATION H* : 1-(2-Chloroéthyl)-3-isopropényl-2-oxo-(3H)imidazo[5,4-b]pyridine**

*Stade A : 3-Isopropényl-2-oxo-(3H)imidazo[5,4-b]pyridine*

Le produit attendu est obtenu selon le procédé décrit dans la préparation C à partir de 2,3-diaminopyridine et d'acétoacétate d'éthyle et après séparation des deux isomères de position par chromatographie sur colonne de silice en utilisant comme éluant un mélange dichlorométhane/éthanol (98/2).

*Stade B : 1-(2-Chloroéthyl)-3-isopropényl-2-oxo-(3H)imidazo[5,4-b]pyridine*

Le produit attendu est obtenu selon le procédé décrit dans la préparation A. L'extraction finale est réalisée avec de l'acétate d'éthyle.

***PREPARATION I* : 1-(2-Chloroéthyl)-3-(1-phénylvinyl)-2(3H)-benzimidazolone**

*Stade A : 3-(1-Phénylvinyl)-2(3H)-benzimidazolone*

100 mmoles de 2-aminoaniline et 100 mmoles de benzoylacétate d'éthyle sont chauffées à 200°C pendant 5 minutes. 100 ml de xylène sont alors ajoutés au mélange précédent et le mélange eau/éthanol est récupéré à l'aide d'un appareil Dean-Stark. Après refroidissement, le produit attendu cristallise ; il est filtré, lavé au cyclohexane et séché.

*Point de fusion : 168-170° C*

*Stade B : 1-(2-Chloroéthyl)-3-(1-phénylvinyl)-2(3H)-benzimidazolone*

A 50 mmoles du composé décrit au stade précédent, dans 150 ml de diméthylformamide sont ajoutées 100 mmoles de carbonate de potassium et 200 mmoles de 1-bromo-2-chloroéthane. Le mélange est chauffé 24 heures à 90°C. Après concentration, le résidu est repris par de l'eau et extrait à l'acétate d'éthyle. La phase organique est lavée par de la potasse 2N puis à l'eau, séchée et évaporée. Le produit attendu est obtenu après purification du résidu par chromatographie sur colonne de silice en utilisant comme éluant un mélange dichlorométhane/éthanol (99/1).

***PREPARATION J* : 1-(2-Chloroéthyl)-3-[1-(trifluorométhyl)vinyl]-2(3H)benzimidazolone**

*Stade A : 3-[1-(trifluorométhyl)vinyl]-2(3H)-benzimidazolone*

Le produit attendu est obtenu selon le procédé décrit dans la préparation C à partir de 2-aminoaniline et de trifluoroacétylacétate d'éthyle.

*Point de fusion : 138-140° C*

*Stade B : 1-(2-Chloroéthyl)-3-[1-(trifluorométhyl)vinyl]-2(3H)-benzimidazolone*

Le produit attendu est obtenu selon le procédé décrit dans la préparation A. L'extraction finale est réalisée avec de l'acétate d'éthyle.

*Point de fusion : 158-160° C*

***PREPARATION K* : 1-(2-Chloroéthyl)-3-(sec-but-1-ènyl)-2(3H)-benzimidazolone**

*Stade A : 3-(sec-But-1-ènyl)-2(3H)-benzimidazolone*

Le produit attendu est obtenu selon le procédé décrit dans la préparation B à partir de 2-aminoaniline et de propionylacétate de méthyle.

*Stade B : 1-(2-Chloroéthyl)-3-(sec-but-1-ènyl)-2(3H)-benzimidazolone*

Le produit attendu est obtenu selon le procédé décrit dans la préparation A.

***PREPARATION L* : 1-(2-Chloroéthyl)-3-isopropényl-2-oxo-(3H)-imidazo[4,5-b]pyridine**

Le produit attendu est obtenu selon le procédé décrit dans la préparation H après séparation au stade A des deux isomères de position.

***PREPARATION M* : 1-(2-Chloroéthyl)-3-(sec-but-2-ènyl)-2(3H)-benzimidazolone**

*Stade A : 3-(sec-But-2-ènyl)-2(3H)-benzimidazolone*

Le produit attendu est obtenu selon le procédé décrit dans la préparation B à partir de 2-aminoaniline et de 2-méthylacétoacétate d'éthyle.

*Point de fusion : 95-97° C*

*Stade B : 1-(2-Chloroéthyl)-3-(sec-but-2-ènyl)-2(3H)-benzimidazolone*

Le produit attendu est obtenu selon le procédé décrit dans la préparation A.

***PREPARATION N* : Mélange de 3-isopropényl-5-trifluorométhyl-2(3H)benzimidazolone et de 3-isopropényl-6-trifluorométhyl-2(3H)-benzimidazolone**

Le mélange d'isomères de position est obtenu selon le procédé décrit dans la préparation B à partir de 2-amino-4-trifluorométhylaniline et d'acétoacétate de méthyle.

**<u>EXEMPLE 1</u> : 1-{2-[4-(N-Propionyl-(3,4-dichlorophényl)amino)pipéridino]éthyl}-3-isopropényl-2(3H)-benzimidazolone**

*Stade A : 1-Benzyl-4-[(3,4-Dichlorophényl)amino]pipéridine*

190 mmoles de 1-benzyl-4-pipéridone, 240 mmoles de 3,4-dichloroaniline, 0,02 g d'APTS et 200 ml de toluène sont placés dans un ballon équipé d'un réfrigérant et d'un appareil Dean Stark. L'ensemble est porté 24 heures à reflux. Après évaporation du solvant, le résidu est repris par 500 ml de méthanol et 450 mmoles de borohydrure de sodium sont ajoutées progressivement. L'ensemble est maintenu 2 jours sous agitation. Le produit attendu est obtenu après concentration par filtration du précipité formé.

*Point de fusion : 95°C*

*Stade B : 1-Benzyl-4-[N-propionyl-(3,4-dichlorophényl)amino]pipéridine*

12 mmoles du composé obtenu au stade précédent et 32 mmoles d'anhydride propionique sont placés dans 60 ml de xylène anhydre. L'ensemble est porté à reflux 20 heures. Après refroidissement, la solution est traitée par une solution aqueuse ammoniacale (0.5 N). La phase organique est ensuite lavée à l'eau jusqu'à neutralisation, séchée et évaporée et conduit au produit attendu.

*Stade C : 4-[N-Propionyl-(3,4-dichlorophényl)amino]pipéridine*

4,2 mmoles du composé obtenu au stade précédent dans 25 ml de dichlorométhane sont débenzylées en présence de 4,6 mmoles d'α-chloroéthylchloroformiate selon le procédé décrit dans J.O.C., <u>49</u>, 2081-2082, 1984.

*Stade D : 1-{2-[4-(N-Propionyl-(3,4-dichlorophényl)amino)pipéridino]éthyl}-3-isopropényl-2(3H)-benzimidazolone*

4 mmoles du composé obtenu au stade précédent et 4 mmoles du composé décrit dans la préparation A sont placées dans 30 ml de diméthylformamide en présence de 4,4 mmoles de carbonate de potassium. L'ensemble est agité à 100°C pendant 18 heures. Après refroidissement et filtration, le solvant est évaporé. Le résidu est repris par un mélange dichlorométhane/eau. La phase organique est lavée jusqu'à neutralité, séchée et évaporée. Le produit attendu est obtenu après purification du résidu par chromatographie sur colonne de silice en utilisant comme éluant un mélange dichlorométhane/éthanol (95/5).

*Point de fusion : 156°C*

| *Microanalyse élémentaire :* | | | | |
|---|---|---|---|---|
| | *C %* | *H %* | *N %* | *S %* |
| *calculé* | *62,28* | *6,03* | *11,17* | *14,14* |
| *trouvé* | *61,79* | *6,08* | *10,85* | *13,83* |

**<u>EXEMPLE 2</u> : 1-{2-[4-(N-Propionylanilino)pipéridino]éthyl}-3-isopropényl-2(3H)-benzimidazolone**

*Stade A : 1-(2-Hydroxyéthyl)-4-(N-propionylanilino)pipéridine*

A 100 mmoles de 4-(N-propionylanilino)pipéridine en solution dans 100 ml de méthanol anhydre refroidi à 15°C, est ajoutée, lentement, une solution refroidie à 15°C contenant 100 mmoles d'oxyde d'éthylène dans du toluène. Après retour à température ambiante et élimination de l'azéotrope formé par distillation, on obtient le produit attendu après évaporation à sec.

*Stade B : 1-(2-Chloroéthyl-4-(N-propionylanilino)pipéridine*

A 100 mmoles du composé obtenu au stade précédent dans 200 ml de toluène sont ajoutées 125 mmoles de chlorure de thionyle en solution dans 30 ml de toluène. L'ensemble est porté 2 heures à reflux. Après refroidissement, le précipité formé est filtré, lavé au toluène puis à l'éther et séché et conduit au produit attendu.

*Stade C : 1-{2-[4-(N-Propionylanilino)pipéridino]éthyl}-3-isopropényl-2(3H)-benzimidazolone*

7 mmoles du produit obtenu au stade précédent, 7 mmoles du composé décrit dans la préparation B et 2,1 g de carbonate de potassium dans 60 ml de diméthylformamide sont portés à 85°C pendant 20 heures. Après refroidissement et évaporation du solvant, le résidu est repris par de l'acétate d'éthyle et lavé à l'eau. Après évaporation de la phase organique, le produit attendu est obtenu après purification du résidu par chromatographie sur colonne de silice en utilisant comme éluant un mélange dichlorométhane/acétate d'éthyle (50/50).

*Point de fusion : 136° C*

| *Microanalyse élémentaire :* | | | |
|---|---|---|---|
| | *C %* | *H %* | *N %* |
| calculé | *72,19* | *7,46* | *12,95* |
| trouvé | *72,12* | *7,80* | *12,78* |

**<u>EXEMPLE 3</u> : 1-{2-[4-(N-Butyrylanilino)pipéridino]éthyl}-3-isopropényl-2(3H)-benzimidazolone**

Le produit attendu est obtenu selon le procédé décrit dans l'exemple 2 en remplaçant au stade A la 4-(N-propionylanilino)pipéridine par la 4-(N-butyrylanilino)pipéridine.

| *Microanalyse élémentaire:* | | | |
|---|---|---|---|
| | *C %* | *H %* | *N %* |
| *calculé* | *72,62* | *7,67* | *12,55* |
| *trouvé* | *72,64* | *7,69* | *12,31* |

**<u>EXEMPLE 4</u> : 1-{2-[4-(N-Propionylanilino)pipéridino]éthyl}-3-cyclopent-1-ènyl-2(3H)-benzimidazolone**

Le produit attendu est obtenu selon le procédé décrit dans l'exemple 2 en utilisant au stade C le produit décrit dans la préparation C.

| *Microanalyse élémentaire :* | | | |
|---|---|---|---|
| | *C %* | *H %* | *N %* |
| *calculé* | *73,33* | *7,47* | *12,22* |
| *trouvé* | *72,86* | *7,44* | *11,98* |

**<u>EXEMPLE 5</u> : 1-{2-[4-(N-Propionylanilino)pipéridino]éthyl}-3-isopropényl-2(3H)-naphto[2,3-d]imidazolone**

Le produit attendu est obtenu selon le procédé décrit dans l'exemple 2 en utilisant au stade C le produit décrit dans la préparation D.

*Point de fusion : 173-175° C*

| *Microanalyse élémentaire :* | | | |
|---|---|---|---|
| | *C %* | *H %* | *N %* |
| *calculé* | *74,66* | *7,10* | *11,61* |
| *trouvé* | *74,93* | *7,04* | *11,41* |

**<u>EXEMPLE 6</u> : 1-{2-[4-(N-Propionylanilino)pipéridino]éthyl}-3-isopropényl-5,6-dichloro-2(3H)-benzimidazolone**

Le produit attendu est obtenu selon le procédé décrit dans l'exemple 2 en utilisant au stade C le produit décrit dans la préparation E.

| *Microanalyse élémentaire :* | | | | |
|---|---|---|---|---|
| | *C %* | *H %* | *N %* | *Cl%* |
| *calculé* | *62,28* | *6,03* | *11,17* | *14,14* |
| *trouvé* | *61,95* | *5,97* | *10,66* | *14,49* |

**EXEMPLE 7 : 1-{2-[4-(N-Propionylanilino)pipéridino]éthyl}-3-isopropényl-7-méthyl-2(3H)-benzimidazolone**

Le produit attendu est obtenu selon le procédé décrit dans l'exemple 2 en utilisant au stade C le produit décrit dans la préparation F.

| *Microanalyse élémentaire :* | | | |
|---|---|---|---|
| | *C %* | *H %* | *N %* |
| *calculé* | *72,62* | *7,67* | *12,55* |
| *trouvé* | *71,85* | *7,79* | *12,43* |

**EXEMPLE 8 : 1-{2-[4-(N-Propionylanilino)pipéridino]éthyl}-3-isopropényl-4-nitro-2(3H)-benzimidazolone**

Le produit attendu est obtenu selon le procédé décrit dans l'exemple 2 en utilisant au stade C le produit décrit dans la préparation G.

Spectre de masse : FAB-$[M+H]^+$ : m/z = 478

**EXEMPLE 9 : 1-{2-[4-(N-Propionyl-(3-méthoxyphényl)amino)pipéridino]éthyl}-3-isopropényl-2(3H)-benzimidazolone**

Le produit attendu est obtenu selon le procédé décrit dans l'exemple 1 en utilisant au stade A la 3-méthoxyaniline.

| *Microanalyse élémentaire :* | | | |
|---|---|---|---|
| | *C %* | *H %* | *N %* |
| *calculé* | *70,10* | *7,41* | *12,11* |
| *trouvé* | *70,17* | *7,44* | *12,04* |

**EXEMPLE 10 : 1-{2-[4-(N-Propionyl-(4-méthylphényl)amino)pipéridino]éthyl}-3-isopropényl-2(3H)-benzimidazolone**

Le produit attendu est obtenu selon le procédé décrit dans l'exemple 1 en utilisant au stade A la 4-méthylaniline.

*Point de fusion : 110°C*

| *Microanalyse élémentaire :* | | | |
|---|---|---|---|
| | *C %* | *H %* | *N %* |
| *calculé* | *72,62* | *7,67* | *12,55* |
| *trouvé* | *72,62* | *7,70* | *12,28* |

**EXEMPLE 11 : 1-{2-{4-(N-Propionyl-(4-chlorophényl)amino)pipéridino]éthyl}-3-isopropényl-2(3H)-benzimidazolone**

Le produit attendu est obtenu selon le procédé décrit dans l'exemple 1 en utilisant au stade A la 4-chloroaniline.

*Point de fusion : 128°C*

| *Microanalyse élémentaire :* | | | | |
|---|---|---|---|---|
| | *C %* | *H %* | *N %* | *Cl %* |
| *calculé* | *66,87* | *6,69* | *12,00* | *7,59* |
| *trouvé* | *66,72* | *6,72* | *11,81* | *7,96* |

**EXEMPLE 12 : 1-{2-[4-(N-Propionyl-(3-chlorophényl)amino)pipéridino]éthyl}-3-isopropényl-2(3H)-benzimidazolone**

Le produit attendu est obtenu selon le procédé décrit dans l'exemple 1 en utilisant au stade A la 3-chloroaniline.
*Point de fusion : 128° C*

| *Microanalyse élémentaire :* | | | | |
|---|---|---|---|---|
| | *C %* | *H %* | *N %* | *Cl %* |
| *calculé* | *66,87* | *6,69* | *12,00* | *7,59* |
| *trouvé* | *66,59* | *6,65* | *11,66* | *7,68* |

**EXEMPLE 13 : 1-{2-[4-(N-Propionyl-(2-naphtyl)amino)pipéridino]éthyl}-3-isopropényl-2(3H)-benzimidazolone**

Le produit attendu est obtenu selon le procédé décrit dans l'exemple 1 en utilisant au stade A la 2-naphtylamine.
*Point de fusion : 118-120° C*

| *Microanalyse élémentaire :* | | | |
|---|---|---|---|
| | *C %* | *H %* | *N %* |
| *calculé* | *74,66* | *7,10* | *11,61* |
| *trouvé* | *74,13* | *6,94* | *11,50* |

**EXEMPLE 14 : 1-{2-[4-(N-Propionyl-(4-fluorophényl)amino)pipéridino]éthyl}-3-isopropényl-2(3H)-benzimidazolone**

Le produit attendu est obtenu selon le procédé décrit dans l'exemple 1 en utilisant au stade A la 4-fluoroaniline.

| *Microanalyse élémentaire :* | | | |
|---|---|---|---|
| | *C %* | *H %* | *N %* |
| *calculé* | *69,31* | *6,94* | *12,43* |
| *trouvé* | *69,51* | *6,86* | *12,13* |

**EXEMPLE 15 : 1-{2-[4-(N-Propionyl-(4-(1-hydroxy-2,2,2-trifluoroéthyl)phényl)amino) pipéridino]éthyl}-3-isopropényl-2(3H)-benzimidazolone**

Le produit attendu est obtenu selon le procédé décrit dans l'exemple 1 en utilisant au stade A le 4-(1-hydroxy-2,2,2-trifluoroéthyl)aniline.

| *Microanalyse élémentaire :* | | | |
|---|---|---|---|
| | *C %* | *H %* | *N %* |
| *calculé* | *63,38* | *6,27* | *10,56* |
| *trouvé* | *63,39* | *6,25* | *10,31* |

**<u>EXEMPLE 16</u> : 1-{2-[4-(N-Propionyl-(3,4-dichlorophényl)amino)pipéridino]éthyl}-3-isopropényl-2-oxo-(3H)-imidazo[5,4-b]pyridine**

Le produit attendu est obtenu selon le procédé décrit dans l'exemple 1 en utilisant au stade D le produit décrit dans la préparation H.

<u>Spectre de masse</u> : ionisation chimique/$NH_3$ : $[M+H]^+$ : m/z = 503

**<u>EXEMPLE 17</u> : 1-{2-[4-(N-Propionyl-(3,4-dichlorophényl)amino)pipéridino]éthyl}-3-cyclopentèn-1-yl-2(3H)-benzimidazolone**

Le produit attendu est obtenu selon le procédé décrit dans l'exemple 2 en utilisant au stade A la 4-[N-propionyl-(3,4-dichlorophényl)amino]pipéridine et au stade C le produit décrit dans la préparation C.

*Point de fusion : 155-157° C*

| *Microanalyse élémentaire :* | | | | |
|---|---|---|---|---|
| | *C %* | *H %* | *N %* | *Cl %* |
| *calculé* | *63,76* | *6,11* | *10,62* | *13,36* |
| *trouvé* | *63,29* | *6,02* | *10,17* | *13,44* |

**<u>EXEMPLE 18</u> : 1-{2-[4-(N-Propionyl-(3,4-dichlorophényl)amino)pipéridino]éthyl}-3-isopropényl-2(3H)-naphto[2,3-d]imidazolone**

Le produit attendu est obtenu selon le procédé décrit dans l'exemple 2 en utilisant au stade A la 4-[N-propionyl-(3,4-dichlorophényl)amino]pipéridine et au stade C le produit décrit dans la préparation D.

*Point de fusion : 185-190° C*

| *Microanalyse élémentaire :* | | | | |
|---|---|---|---|---|
| | *C %* | *H %* | *N %* | *Cl %* |
| *calculé* | *65,33* | *5,85* | *10,16* | *12,86* |
| *trouvé* | *64,72* | *5,89* | *9,96* | *13,62* |

**<u>EXEMPLE 19</u> : 1-{2-[4-[N-(Pipéridinocarbonyl)anilino]pipéridino]éthyl}-3-isopropényl-2(3H)-benzimidazolone**

*Stade A : 1-Benzyl-4-[N-(pipéridinocarbonyl)anilino]pipéridine*

A 200 mmoles d'une solution de phosgène à 20 % refroidie à 10°C, sont ajoutées, lentement, 60 mmoles de 1-benzyl-4-anilinopipéridine dans 50 ml de toluène. L'ensemble est agité 30 minutes puis porté à 80°C pendant 2 heures. Le précipité formé est essoré, lavé au toluène puis à l'oxyde d'isopropyle. A 58 mmoles de pipéridine dans 150 ml de toluène, on ajoute 52 mmoles du précipité obtenu précédemment et le mélange est chauffé à 80°C. Après refroidissement, l'ensemble est versé sur 200 ml d'eau. La phase organique lavée à l'eau est alors séchée et évaporée et conduit au produit attendu qui cristallise.

*Point de fusion : 118-119° C*

*Stade B: 4-[N-(Pipéridinocarbonyl)anilino]pipéridine*

A 50 mmoles du composé obtenu au stade précédent dans 200 ml de méthanol, sont ajoutées 100 mmoles de formiate d'ammonium et 1,8 g de Palladium/charbon. Le mélange est porté 2 heures à reflux. Le catalyseur est filtré et le filtrat évaporé. Le résidu est alors repris par 150 ml d'acide chlorhydrique 1N. La phase aqueuse acide est lavée à l'éther puis alcalinisée par de la soude. Après extraction à l'éther, lavage de la phase éthérée par une solution saturée de chlorure de sodium, filtration et évaporation, on obtient le produit attendu sous forme d'un solide blanc.

*Point de fusion : 125° C*

*Stade C : 1-{2-[4-[N-(Pipéridinocarbonyl)anilino]pipéridino]éthyl}-3-isopropényl-2(3H)-benzimidazolone*

Le produit attendu est obtenu selon le procédé décrit au stade D de l'exemple 1 à partir du composé décrit au

stade précédent.

| *Microanalyse élémentaire :* | | | |
|---|---|---|---|
| | *C %* | *H %* | *N %* |
| *calculé* | *71,43* | *7,65* | *14,36* |
| *trouvé* | *70,91* | *7,78* | *13,64* |

**EXEMPLE 20 : 1-{2-[4-(N-Méthoxyacétyl-(3,4-dichlorophényl)amino)pipéridino]éthyl}-3-isopropényl-2(3H)-benzimidazolone**

*Stade A :* Ce stade est identique au stade A de l'exemple 1.

*Stade B : 1-Benzyl-4-[N-méthoxyacétyl-(3,4-dichlorophényl)amino]pipéridine*

A 12 mmoles de 1-benzyl-4-[(3,4-dichlorophényl)amino]pipéridine obtenu au stade précédent dans 50 ml de tétrahydrofurane (THF) anhydre sont ajoutées 12 mmoles de chlorure de méthoxyacétyle. Le mélange est porté 3 heures à reflux. Après refroidissement, le produit attendu est obtenu par filtration du précipité qui est lavé au THF et séché.

*Stade C : 4-[N-méthoxyacétyl-(3,4-dichlorophényl)amino]pipéridine*

Le produit attendu est obtenu selon le procédé décrit au stade C de l'exemple 1.

*Stade D : 1-{2-[4-(N-Méthoxyacétyl-(3,4-dichlorophényl)amino)pipéridino]éthyl}-3-isopropényl-2(3H)-benzimidazolone*

Le produit attendu est obtenu selon le procédé décrit au stade D de l'exemple 1.

| *Microanalyse élémentaire :* | | | | |
|---|---|---|---|---|
| | *C %* | *H %* | *N %* | *Cl %* |
| *calculé* | *60,35* | *5,84* | *10,83* | *13,70* |
| *trouvé* | *61,00* | *6,13* | *9,99* | *13,98* |

**EXEMPLE 21 : 1-{2-[4-(N-Propionylanilino)pipéridino]éthyl}-3-(1-phénylvinyl)-2(3H)-benzimidazolone**

Le produit attendu est obtenu selon le procédé décrit dans l'exemple 1 en utilisant au stade A l'aniline et au stade D le produit décrit dans la préparation I.

| *Microanalyse élémentaire :* | | | |
|---|---|---|---|
| | *C %* | *H %* | *N %* |
| *calculé* | *75,28* | *6,93* | *11,33* |
| *trouvé* | *74,45* | *6,82* | *10,91* |

**EXEMPLE 22 : 1-{2-[4-(N-Propionyl-(3,4-dichlorophényl)amino)pipéridino]éthyl}-3-[1-(trifluorométhyl)vinyl]-2(3H)-benzimidazolone**

Le produit attendu est obtenu selon le procédé décrit dans l'exemple 1 en utilisant au stade D le produit décrit dans la préparation J.

Spectre de masse : Ionisation Chimique ($NH_3$) : $[M+H]^+$ : m/z = 555

**EXEMPLE 23 : 1-{2-[4-(N-Propionyl-(3-chloro-4-méthylphényl)amino)pipéridino] éthyl}-3-isopropényl-2(3H)-benzimidazolone**

Le produit attendu est obtenu selon le procédé décrit dans l'exemple 1 en utilisant au stade A la 3-chloro-4-méthylaniline.

*Point de fusion : 138-140° C*

| *Microanalyse élémentaire :* | | | | |
|---|---|---|---|---|
| | *C %* | *H %* | *N %* | *Cl %* |
| *calculé* | *67,42* | *6,91* | *11,65* | *7,37* |
| *trouvé* | *67,67* | *6,88* | *11,41* | *7,56* |

**EXEMPLE 24 : 1-{2-[4-(N-Propionyl-(3,4-diméthylphényl)amino)pipéridino]éthyl}-3-isopropényl-2(3H)-benzimidazolone**

Le produit attendu est obtenu selon le procédé décrit dans l'exemple 1 en utilisant au stade A la 3,4-diméthylaniline et en réalisant au stade C une hydrogénation catalytique en présence de Palladium/charbon comme catalyseur.
*Point de fusion : 138° C*

| *Microanalyse élémentaire :* | | | |
|---|---|---|---|
| | *C %* | *H %* | *N %* |
| *calculé* | *73,01* | *7,88* | *12,16* |
| *trouvé* | *73,25* | *7,81* | *11,95* |

**EXEMPLE 25 : 1-{2-[4-(N-Propionyl-(3,4-difluorophényl)amino)pipéridino]éthyl}1-3-isopropényl-2(3H)-benzimidazolone**

Le produit attendu est obtenu selon le procédé décrit dans l'exemple 1 en utilisant au stade A la 3,4-difluoroaniline.
*Point de fusion : 166° C*

| *Microanalyse élémentaire :* | | | |
|---|---|---|---|
| | *C %* | *H %* | *N %* |
| *calculé* | *66,65* | *6,45* | *11,96* |
| *trouvé* | *66,18* | *6,42* | *11,66* |

**EXEMPLE 26 : 1-{2-[4-(N-Propionyl-(3,4-dichlorophényl)amino)pipéridino]éthyl}-3(sec-but-1-ènyl)-2(3H)-benzimidazolone**

Le produit attendu est obtenu selon le procédé décrit dans l'exemple 1 en utilisant au stade D le procédé décrit dans la préparation K.
*Point de fusion : 131° C*

| *Microanalyse élémentaire :* | | | | |
|---|---|---|---|---|
| | *C %* | *H %* | *N %* | *Cl %* |
| *calculé* | *62,91* | *6,26* | *10,87* | *13,76* |
| *trouvé* | *62,19* | *6,27* | *10,35* | *13,62* |

**EXEMPLE 27 : 1-{2-[4-(N-Acétyl-(3,4-dichlorophényl)amino)pipéridino]éthyl}-3-isopropényl-2(3H)-benzimidazolone**

Le produit attendu est obtenu selon le procédé décrit dans l'exemple 1 en utilisant au stade B l'anhydride acétique.
*Point de fusion : 174° C*

| *Microanalyse élémentaire :* | | | | |
|---|---|---|---|---|
| | *C %* | *H %* | *N %* | *Cl %* |
| *calculé* | *61,60* | *5,79* | *11,49* | *14,55* |

(suite)

| *Microanalyse élémentaire :* | | | | |
|---|---|---|---|---|
| | *C %* | *H %* | *N %* | *Cl %* |
| *trouvé* | *61,73* | *5,88* | *11,16* | *14,23* |

**EXEMPLE 28 : 1-{2-[4-(N-Propionyl-(3,4-dichlorophényl)amino)pipéridino]éthyl}-3-isopropényl-2-oxo-(3H)-imidazo[4,5-b]pyridine**

Le produit attendu est obtenu selon le procédé décrit dans l'exemple 1 en utilisant au stade D le produit décrit dans la préparation L.

| *Microanalyse élémentaire :* | | | | |
|---|---|---|---|---|
| | *C %* | *H %* | *N %* | *Cl %* |
| *calculé* | *59,76* | *5,82* | *13,94* | *14,11* |
| *trouvé* | *59,19* | *5,85* | *13,28* | *13,85* |

**EXEMPLE 29 : 1-{2-[4-(N-Propionyl-(3,4-dichlorophényl)amino)pipéridino]éthyl}-3-(sec-but-2-ènyl)-2(3H)-benzimidazolone**

Le produit attendu est obtenu selon le procédé décrit dans l'exemple 1 en utilisant au stade D le produit décrit dans la préparation M.

Spectre de masse : Ionisation chimique ($NH_3$) : $[M+H]^+$ : m/z = 516

**EXEMPLE 30 : 1-{2-[4-(N-Propionyl-(3,4-diméthoxyphényl)amino)pipéridino]éthyl}-3-isopropényl-2(3H)-benzimidazolone**

Le produit attendu est obtenu selon le procédé décrit dans l'exemple 1 en utilisant au stade A la 3,4-diméthoxyaniline.

*Point de fusion : 145° C*

| *Microanalyse élémentaire :* | | | |
|---|---|---|---|
| | *C %* | *H %* | *N %* |
| *calculé* | *68,11* | *7,11* | *10,97* |
| *trouvé* | *68,27* | *7,37* | *11,37* |

**EXEMPLE 31 : 1-{2-[4-(N-Propionyl-(pyrid-4-yl)amino)pipéridino]éthyl}-3-isopropényl-2(3H)-benzimidazolone**

Le produit attendu est obtenu selon le procédé décrit dans l'exemple 1 en utilisant au stade A la 4-aminopyridine.

*Point de fusion : 106° C*

| *Microanalyse élémentaire :* | | | |
|---|---|---|---|
| | *C %* | *H %* | *N %* |
| *calculé* | *69,26* | *7,21* | *16,15* |
| *trouvé* | *69,33* | *7,26* | *16,00* |

**<u>EXEMPLE 32</u> : 1-{2-[4-(N-Propionyl-(3,4-dichlorophényl)amino)pipéridino]éthyl}-3-isopropényl-7-trifluorométhyl-2(3H)-benzimidazolone**

| *Microanalyse élémentaire :* | | | | |
|---|---|---|---|---|
| | *C %* | *H %* | *N %* | *Cl %* |
| *calculé* | *56,95* | *5,13* | *9,84* | *12,45* |
| *trouvé* | *57,03* | *5,21* | *9,59* | *12,71* |

**<u>EXEMPLE 33</u> : 1-{2-[4-(N-Propionyl-(3,4-dichlorophényl)amino)pipéridino]éthyl}-3-isopropényl-5-trifluorométhyl-2(3H)-benzimidazolone**

et

**<u>EXEMPLE 34</u> : 1-{2-[4-(N-Propionyl-(3,4-dichlorophényl)amino)pipéridino]éthyl}-3-isopropényl-6-trifluorométhyl-2(3H)-benzimidazolone**

Le mélange de composés a été obtenu selon le procédé décrit dans l'exemple 2 en utilisant au stade C le produit décrit dans la préparation N.
Les deux composés ont alors été séparés par chromatographie sur colonne de silice en utilisant comme éluant un mélange dichlorométhane/éthanol (98/2).
La position des groupements trifluorométhyle a été déterminée par RMN du proton.
Exemple 33 : Présence d'un singulet sur le noyau phényle situé à 7,25 ppm.
Exemple 34 : Présence d'un singulet sur le noyau phényle situé à 7,60 ppm.

**<u>EXEMPLE 35</u> : Iodure de 1-méthyl-1-[2-(3-isopropényl-2-oxo-(3H)-benzimidazol-1-yl)éthyl]-4-[N-propionyl-(3,4-dichlorophényl)amino]pipéridinium**

424 mmoles du composé décrit dans l'exemple 1 et 500 mmoles d'iodure de méthyle sont agitées dans 25 ml d'acétone pendant 5 jours. Le produit attendu est obtenu par filtration du précipité, dissolution dans l'eau et lyophilisation.

<u>Spectre de masse</u> : FAB : $[M-I]^+$ : m/z = 515

**<u>EXEMPLE 36</u> : Iodure de 1-méthyl-1-[2-(3-isopropényl-2-oxo-(3H)-benzimidazol-1-yl)éthyl]-4-(N-propionylanilino)pipéridinium**

Le produit attendu est obtenu selon le procédé décrit dans l'exemple 35 à partir du composé décrit dans l'exemple 2.

| *Microanalyse élémentaire :* | | | | |
|---|---|---|---|---|
| | *C %* | *H %* | *N %* | *I %* |
| *calculé* | *56,45* | *6,14* | *9,75* | *22,09* |
| *trouvé* | *56,10* | *6,14* | *9,56* | *22,16* |

**<u>EXEMPLE 37</u> : 1-{2-[4-(N-Phtalimidoacétyl-(3-chloro-4-méthylphényl)amino) pipéridino]éthyl}-3-isopropényl-2 (3H)-benzimidazolone**

Le produit attendu est obtenu selon le procédé décrit dans l'exemple 1 en utilisant les produits de départ correspondants.

| *Microanalyse élémentaire :* | | | | |
|---|---|---|---|---|
| | *C %* | *H %* | *N %* | *Cl %* |
| *calculé* | *66,71* | *5,60* | *11,44* | *5,79* |
| *trouvé* | *66,62* | *5,96* | *10,70* | *5,39* |

**EXEMPLE 38 : 1-{2-[4-(N-Propionyl-(3,4-dichlorophényl)amino)pipéridino]éthyl}-3-(1-phénylvinyl)-2(3H)-benzimidazolone**

Le produit attendu est obtenu selon le procédé décrit dans l'exemple 1 en utilisant au stade A la 3,4-dichloroaniline et au stade D le produit décrit dans la préparation I.

| *Microanalyse élémentaire :* | | | | |
|---|---|---|---|---|
| | *C %* | *H %* | *N %* | *Cl %* |
| *calculé* | *66,07* | *5,72* | *9,94* | *12,58* |
| *trouvé* | *65,30* | *5,62* | *9,34* | *12,34* |

**EXEMPLE 39 : 1-{2-[4-(N-Méthoxyacétyl-(3,4-dichlorophényl)amino)pipéridino]éthyl}-3-cyclopent-1-ènyl-2(3H)-benzimidazolone**

Le produit attendu est obtenu selon le procédé décrit dans l'exemple 2 en utilisant au stade C le produit décrit dans la préparation C.

*Point de fusion : 144-145° C*

| *Microanalyse élémentaire :* | | | | |
|---|---|---|---|---|
| | *C %* | *H %* | *N %* | *Cl %* |
| *calculé* | *61,88* | *5,93* | *10,31* | *13,05* |
| *trouvé* | *62,08* | *6,09* | *9,95* | *12,99* |

**EXEMPLE 40 : 1-{2-[4-(N-Pipéridinocarbonyl-(3,4-dichlorophényl)amino)pipéridino] éthyl}-3-isopropényl-2(3H)-benzimidazolone**

Le produit attendu est obtenu selon le procédé décrit dans l'exemple 1 en utilisant les produits de départ correspondants.

| *Microanalyse élémentaire :* | | | |
|---|---|---|---|
| | *C %* | *H %* | *N %* |
| *calculé* | *62,59* | *6,34* | *12,58* |
| *trouvé* | *62,65* | *6,60* | *11,87* |

**EXEMPLE 41 : 1-{2-[4-(N-Benzoyl-N-méthylamino)pipéridino]éthyl}-3-isopropényl-2(3H)-benzimidazolone**

Le produit attendu est obtenu selon le procédé décrit dans l'exemple 1 en utilisant les produits de départ correspondants.

*Point de fusion : 120-122° C*

| *Microanalyse élémentaire :* | | | |
|---|---|---|---|
| | *C %* | *H %* | *N %* |
| *calculé* | *71,71* | *7,22* | *13,30* |
| *trouvé* | *70,92* | *7,26* | *13,08* |

**EXEMPLE 42 : 1-{2-[4-(N-Benzoyl-(3,4-dichlorophényl)amino)pipéridino]éthyl}-3-isopropényl-2(3H)-benzimidazolone**

Le produit attendu est obtenu selon le procédé décrit dans l'exemple 1 en utilisant les produits de départ correspondants.

*Point de fusion : 158° C*

| Microanalyse élémentaire : | | | | |
|---|---|---|---|---|
| | C % | H % | N % | Cl % |
| calculé | 65,57 | 5,50 | 10,20 | 12,90 |
| trouvé | 65,15 | 5,55 | 9,82 | 13,37 |

**EXEMPLE 43 : 1-{2-[4-(N-Propionyl-(2,5-dichlorophényl)amino)pipéridino]éthyl}-3-isopropényl-2(3H)-benzimidazolone**

Le produit attendu est obtenu selon le procédé décrit dans l'exemple 1 en utilisant les produits de départ correspondants.

*Point de fusion : 148° C*

| Microanalyse élémentaire : | | | | |
|---|---|---|---|---|
| | C % | H % | N % | Cl % |
| calculé | 62,28 | 6,03 | 11,17 | 14,14 |
| trouvé | 62,06 | 6,05 | 10,95 | 14,28 |

**EXEMPLE 44 : 1-{2-[4-(N-Propionyl-(3-chloro-4-méthoxyphényl)amino)pipéridino] éthyl}-3-isopropényl-2(3H)-benzimidazolone**

Le produit attendu est obtenu selon le procédé décrit dans l'exemple 1 en utilisant les produits de départ correspondants.

*Point de fusion : 150-152° C*

| Microanalyse élémentaire : | | | | |
|---|---|---|---|---|
| | C % | H % | N % | Cl % |
| calculé | 65,25 | 6,69 | 11,27 | 7,13 |
| trouvé | 65,67 | 7,05 | 11,13 | 7,14 |

**EXEMPLE 45 : 1-{2-[4-(N-Propionyl-(3-chloro-4-fluorophényl)amino)pipéridino]éthyl}-3-isopropényl-2(3H)-benzimidazolone**

Le produit attendu est obtenu selon le procédé décrit dans l'exemple 1 en utilisant les produits de départ correspondants.

*Point de fusion : 130-132° C*

| Microanalyse élémentaire : | | | | |
|---|---|---|---|---|
| | C % | H % | N % | Cl % |
| calculé | 64,39 | 6,23 | 11,55 | 7,31 |
| trouvé | 64,24 | 6,26 | 11,45 | 7,34 |

**EXEMPLE 46 : 1-{2-[4-(N-Propionyl-(2,4-dichlorophényl)amino)pipéridino]éthyl}-3-isopropényl-2(3H)-benzimidazolone**

Le produit attendu est obtenu selon le procédé décrit dans l'exemple 1 en utilisant les produits de départ correspondants.

| Microanalyse élémentaire : | | | | |
|---|---|---|---|---|
| | C % | H % | N % | Cl % |
| calculé | 62,28 | 6,03 | 11,47 | 14,14 |

(suite)

| *Microanalyse élémentaire :* | | | | |
|---|---|---|---|---|
| | *C %* | *H %* | *N %* | *Cl %* |
| *trouvé* | *61,59* | *6,12* | *10,88* | *14,69* |

**EXEMPLE 47 : 1-{2-[4-(N-Propionyl-(3,5-dichlorophényl)amino)pipéridino]éthyl}-3-isopropényl-2(3H)-benzimidazolone**

Le produit attendu est obtenu selon le procédé décrit dans l'exemple 1 en utilisant les produits de départ correspondants.

*Point de fusion : 170° C*

| *Microanalyse élémentaire :* | | | | |
|---|---|---|---|---|
| | *C %* | *H %* | *N %* | *Cl %* |
| *calculé* | *62,28* | *6,03* | *11,17* | *14,14* |
| *trouvé* | *61,83* | *5,99* | *11,02* | *14,41* |

**EXEMPLE 48 : 1-{2-[4-(N-Phtalimidoacétyl)-(3,4-dichlorophényl)amino)pipéridino] éthyl}-3-isopropényl-2(3H)-benzimidazolone**

Le produit attendu est obtenu selon le procédé décrit dans l'exemple 1 en utilisant les produits de départ correspondants.

**EXEMPLE 49 : 1-{2-[4-(N-Aminoacétyl-(3,4-dichlorophényl)amino)pipéridino]éthyl}-3-isopropényl-2(3H)-benzimidazolone**

Le produit attendu est obtenu par réaction du composé décrit dans l'exemple 48 avec de l'hydrazine.

*Point de fusion : 162° C*

*Spectre de masse : FAB : $[M+H]^+$ : m/z = 502*

**EXEMPLE 50 : 1-{2-[4-(N-Aminoacétyl-(3-chloro-4-méthylphényl)amino)pipéridino] éthyl}-3-isopropényl-2 (3H)-benzimidazolone**

Le produit attendu est obtenu par réaction du composé décrit dans l'exemple 37 avec de l'hydrazine.

*Point de fusion : 134° C*

| *Microanalyse élémentaire :* | | | | |
|---|---|---|---|---|
| | *C %* | *H %* | *N %* | *Cl %* |
| *calculé* | *64,79* | *6,69* | *14,53* | *7,35* |
| *trouvé* | *63,67* | *6,62* | *14,05* | *7,56* |

**Etude pharmacologique des dérivés de l'invention**

**EXEMPLE 51 : Affinité aux récepteurs humains $NK_1$ et $NK_2$**

L'affinité pour les récepteurs humains $NK_1$ et $NK_2$ a été étudiée sur les lymphoblastes humains IM-9 exprimant spécifiquement le récepteur $NK_1$ comme décrit par D.G. PAYAN et coll. (J. Biol. Chem. 1986, 261, 14321-14329) et sur les cellules CHO-K1 transfectées avec le récepteur $NK_2$ selon le kit "CellPhect transfection" (Pharmacia).

Les composés de l'invention ont montré une excellente affinité spécifique pour les récepteurs $NK_1$. En effet, les Ki des composés de l'invention sont compris entre 0,7 et 11 nM alors que ceux observés pour les récepteurs $NK_2$ sont, pour les plus faibles, de l'ordre de la µmole.

**EXEMPLE 52 : Essais sur le muscle lisse isolé**

Afin d'évaluer l'activité fonctionnelle des composés de l'invention comme antagonistes des neurokinines, trois préparations isolées de muscle lisse ont été utilisées : la veine cave de lapin (VCL), l'artère pulmonaire de lapin sans endothélium (APL) et la veine porte de rat (VPR) dont les réponses contractiles sont respectivement médiées par les récepteurs $NK_1$, $NK_2$ et $NK_3$ comme indiqué par D. JUKIC et coll. (Life Sci. 1991, 49, 1463-1469). Le pouvoir antagoniste des composés de l'invention a été exprimé sous forme de $pA_2$ tel que défini par O. ARUNLAKSHANA et H.O. SCHILD (Brit. J. Pharmacol. 1959, 14, 48-58). Les composés de l'invention ont montré une puissante activité antagoniste vis-à-vis des récepteurs $NK_1$ ($pA_2$ comprises entre 8.25 et 9.30) avec une faible activité pour les récepteurs $NK_2$ et $NK_3$ ($pA_2$ comprises entre 5.00 et 6.75).

**EXEMPLE 53 : Etude du potentiel antinociceptif - Test d'Eddy chez la souris**

En raison de l'implication de la substance P dans la transmission nociceptive au niveau spinal (M. OTSUKA et S. KONISHI, TINS, 6, 317-320, 1983) et plus particulièrement après stimulation thermique (A.W. DUGGAN et coll., Brain Research, 1987, 403, 345-349), l'activité pharmacologique in vivo des composés de l'invention a été recherchée chez la souris sur le test d'hyperalgésie thermique initialement décrit par N.B. EDDY et coll. (J. Pharmacol. Exp. Ther., 1953, 107, 385-393). Ce test a été préalablement utilisé pour la mise en évidence de l'activité antinociceptive de substances antagonistes de Substance P de nature peptidique (M.P. PIERCEY et coll., Brain Research, 1986, 385, 74-85) et non peptidiques (A. LECCI et coll., Neuroscience Letters, 1991, 129, 299-302). La méthodologie expérimentale est basée sur la mesure du temps de réaction à la chaleur déterminé par le léchage des pattes antérieures chez la souris (CDI mâle, Ch. River, 25-30 g) placée sur une plaque métallique chauffée à 55°C. Les animaux ont été traités avec les composés de l'invention par voie intraveineuse, à différents temps avant le passage sur la plaque chauffante. La moyenne des temps de réaction obtenue pour chaque lot traité (12 souris par lot) a été comparée à la moyenne du lot témoin correspondant. Les résultats sont exprimés sous forme d'$ED_{50}$ qui correspond à la dose augmentant de 50 % le temps de réaction. Alors que la substance P administrée par voie intraspinale, accélère la réaction de l'animal, les composés de l'invention ont ralenti ce temps de réaction après injection intraveineuse. Par exemple, le composé de l'exemple 1 a exercé une puissante activité, maximale 10 minutes après son administration i.v. avec une $ED_{50}$ de 0,79 µg/kg.

**EXEMPLE 54 : Etude de l'inhibition de l'extravasation plasmatique induite par la substance P chez le cobaye**

L'effet des composés sur l'extravasation plasmatique provoquée par injection intraveineuse de substance P (1 µg/kg) chez le cobaye a été évalué au niveau de la vessie, selon la méthode décrite chez le rat par C. GARRET et coll. (Proc. Natl. Acad. Sci., USA 1991, 88, 10208-20212). L'accumulation vésicale de bleu Evan's injecté i.v. en même temps que la substance P et 10 minutes avant le sacrifice, a été quantifiée par spectrophotométrie après extraction du colorant par l'acétone. L'activité inhibitrice des composés administrés par voie intraveineuse 5 minutes avant la substance P a été exprimée en % d'inhibition par comparaison à un lot contrôle (8 animaux par lot). A titre d'exemple, le composé de l'exemple 1 a exercé une très bonne activité avec une $ED_{50}$ de 0,16 mg/kg.

**EXEMPLE 55 : Etude de l'inhibition de la bronchoconstriction induite par la substance P chez le cobaye**

L'étude est réalisée sur des cobayes Hartley mâles (Charles River) de poids moyen 300 à 400g. L'étude est réalisée sur des animaux anesthésiés (carbamate d'éthyle 1,5 g/kg) et curarisés (flaxedil 0,2 mg/kg iv) ventilés avec une fréquence de 60 par minute et un volume courant de 10 ml/kg. Les animaux sont pré-traités par pyrilamine (1 mg/kg iv), propranolol (1 mg/kg iv). Le critère de jugement de la bronchoconstriction est l'augmentation de la pression d'insufflation trachéale (PIT) induite par l'injection de substance P par voie iv à la dose de 2 nm/kg, iv, chaque animal étant son propre témoin. L'injection du produit testé s'effectue par rapport au temps T0 injection du produit. Les résultats sont exprimés en pourcentage d'inhibition de la bronchoconstriction induite par la substance P, ce pourcentage étant calculé selon la formule suivante :

$$(\Delta \text{ PIT avant produit} - \Delta \text{ PIT après produit}) / \Delta \text{ PIT avant produit (exprimé en pourcentage)}.$$

Lors de ce test, le composé de l'exemple 9 présente une dose inhibitrice de 50 % à la dose de 0,1 mg/kg iv.

**Composition Pharmaceutique**

**EXEMPLE 56 : Comprimé : formule de préparation pour 1000 comprimés dosés à 2 mg**

| | |
|---|---|
| Composé de l'exemple 1 | 2 g |
| Hydroxypropylcellulose | 2 g |
| Amidon de blé | 10 g |
| Lactose | 100 g |
| Stéarate de magnésium | 3 g |
| Talc | 3 g |

**Revendications**

1. Composé de formule (I):

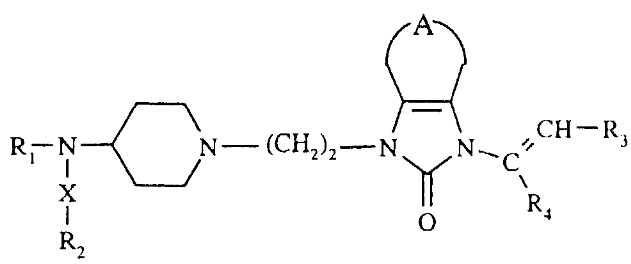

(I)

dans laquelle:

$R_1$ représente un groupement alkyle ($C_1$-$C_6$) linéaire ou ramifié, phényle, naphtyle, pyridyle ou thiényle, chaque groupement phényle, naphtyle, pyridyle ou thiényle étant éventuellement substitué par un ou plusieurs atomes d'halogène ou groupements hydroxy, alkoxy ($C_1$-$C_6$) linéaire ou ramifié, alkyle ($C_1$-$C_6$) linéaire ou ramifié, trihalogénométhyle ou l-hydroxy-2,2,2-trifluoroéthyle,

$R_2$ représente un atome d'hydrogène, un groupement alkyle ($C_1$-$C_6$) linéaire ou ramifié (substitué ou non par un ou plusieurs groupements alkoxy ($C_1$-$C_6$) linéaire ou ramifié ou phényle, amino ou phtalimido), phényle (substitué ou non par un ou plusieurs atomes d'halogène ou groupements alkyle ($C_1$-$C_6$) linéaire ou ramifié, alkoxy ($C_1$-$C_6$) linéaire ou ramifié, hydroxy, trihalogénométhyle), cycloalkyle ($C_3$-$C_7$), pipéridino ou amino (substitué ou non par un ou deux groupements alkyle ($C_1$-$C_6$) linéaire ou ramifié),

X représente un groupement CO ou $SO_2$,

$R_3$ représente un atome d'hydrogène ou un groupement alkyle ($C_1$-$C_6$) linéaire ou ramifié,

$R_4$ représente un groupement alkyle ($C_1$-$C_6$) linéaire ou ramifié, un groupement phényle (substitué ou non par un ou plusieurs atomes d'halogène ou groupements alkyle ($C_1$-$C_6$) linéaire ou ramifié, alkoxy ($C_1$-$C_6$) linéaire ou ramifié, hydroxy ou trihalogénométhyle) ou un groupement trihalogénométhyle,

ou bien, $R_3$ et $R_4$ forment ensemble avec les atomes de carbone qui les portent un groupement cycloalkényl ($C_3$-$C_7$),

A représente, avec les atomes de carbone auxquels il est attaché, un cycle phényle, naphtyle, pyridyle, chaque cycle phényle, naphtyle, pyridyle étant éventuellement substitué par un ou plusieurs atomes d'halogène, ou groupements alkyle ($C_1$-$C_6$) linéaire ou ramifié, alkoxy ($C_1$-$C_6$) linéaire ou ramifié, hydroxy, amino, nitro ou trihalogénométhyle,

leurs isomères, les sels d'ammonium quaternaire de la pipéridine correspondants ainsi que leurs sels d'addition

à un acide pharmaceutiquement acceptable.

**2.** Composé de formule (I) selon la revendication 1 dans laquelle X représente un groupement CO.

**3.** Composé de formule (I) selon la revendication 1 dans laquelle $R_3$ représente un atome d'hydrogène.

**4.** Composé de formule (I) selon la revendication 1 dans laquelle $R_4$ représente un groupement alkyle ($C_1$-$C_6$) linéaire ou ramifié.

**5.** Composé de formule (I) selon la revendication 1 dans laquelle A représente un cycle phényle éventuellement substitué par un ou plusieurs atomes d'halogène, ou groupements alkyle ($C_1$-$C_6$) linéaire ou ramifié, alkoxy ($C_1$-$C_6$) linéaire ou ramifié, hydroxy, amino, nitro ou trihalogénométhyle.

**6.** Composé de formule (I) selon la revendication 1 qui est le 1-{2-[4-(N-propionyl-(3,4-dichlorophényl)amino)pipéridino]éthyl}-3-isopropényl-2(3H)-benzimidazolone.

**7.** Composé de formule (I) selon la revendication 1 qui est le 1-{2-[4-(N-méthoxyacétyl-(3,4-dichlorophényl)amino) pipéridino]éthyl}-3-isopropényl-2(3H)-benzimidazolone.

**8.** Procédé de préparation des composés de formule (I) selon la revendication 1 caractérisé en ce que l'on utilise comme produit de départ une amine de formule (II):

$$R_1 - NH_2 \qquad \text{(II)}$$

dans laquelle $R_1$ a la même signification que dans la formule (I),
que l'on fait réagir sur une pipéridone de formule (III):

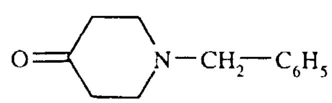

(III)

pour conduire au composé de formule (IV),

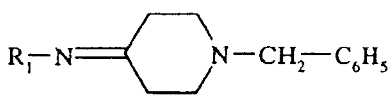

(IV)

dans laquelle $R_1$ a la même signification que dans la formule (I),
qui subit une réduction en présence d'un hydrure métallique,
pour conduire au composé de formule (V) :

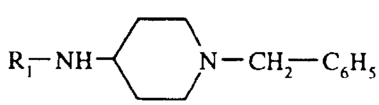

(V)

dans laquelle $R_1$ a la même signification que dans la formule (I),
que l'on met en réaction avec un anhydride, un chlorure d'acide ou le phosgène (suivie d'une réaction avec une amine secondaire),
pour conduire au composé de formule (VI) :

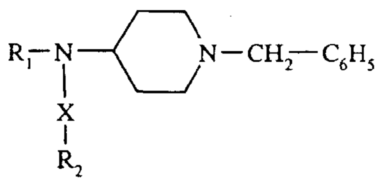

(VI)

dans laquelle $R_1$, $R_2$ et X ont la même signification que dans la formule (I),
qui subit une débenzylation par hydrogénation catalytique, transfert d'hydrogène (en présence de formiate d'ammonium) ou par déalkylation (en présence d'α-chloroformiate de chloréthyle),
pour conduire au composé de formule (VII) :

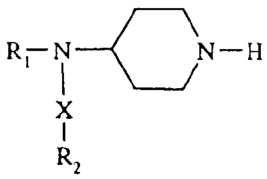

(VII)

dans laquelle $R_1$, $R_2$ et X ont la même signification que dans la formule (I),
que l'on fait réagir avec un composé de formule (VIII) :

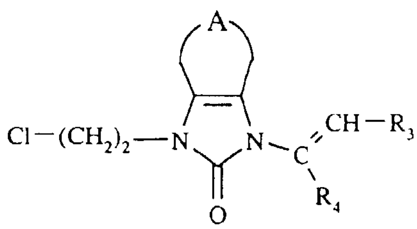

(VIII)

dans laquelle A, $R_3$ et $R_4$ ont la même signification que dans la formule (I),
pour conduire au composé de formule (I),
composé de formule (I) :

- qui peut être, le cas échéant, purifié selon une technique classique de purification,
- dont on sépare, le cas échéant, les isomères selon une technique classique de sépararation,
- que l'on transforme, si on le souhaite, en ses sels d'addition à un acide pharmaceutiquement acceptable ou en sel d'ammonium quaternaire de la pipéridine.

**9.** Procédé de préparation des composés de formule (I) selon la revendication 1 caractérisé en ce que l'on utilise comme produit de départ une pipéridine de formule (IX) :

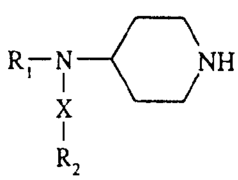

(IX)

dans laquelle $R_1$, $R_2$ et X ont la même signification que dans la formule (I),
que l'on fait réagir avec l'époxyéthane,
pour conduire au composé de formule (X) :

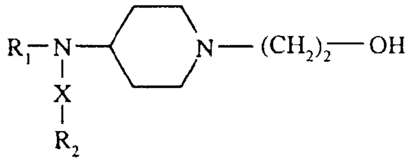

(X)

dans laquelle $R_1$, $R_2$ et X ont la même signification que dans la formule (I), qui subit l'action du chlorure de thionyle pour conduire au composé de formule (XI) :

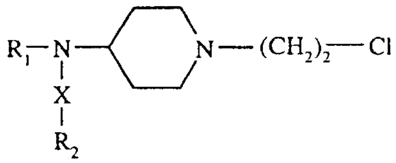

(XI)

dans laquelle $R_1$, $R_2$ et X ont la même signification que dans la formule (I), que l'on fait réagir avec le composé de formule (XII) :

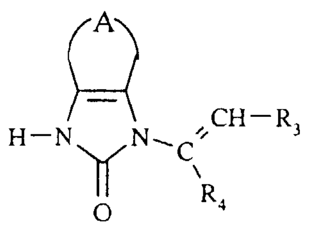

(XII)

dans laquelle A, $R_3$ et $R_4$ sont tels que définis dans la formule (I), pour conduire au composé de formule (I),
composé de formule (I) :

- qui peut être, le cas échéant, purifié selon une technique classique de purification,
- dont on sépare, le cas échéant, les isomères selon une technique classique de sépararation,
- que l'on transforme, si on le souhaite, en ses sels d'addition à un acide pharmaceutiquement acceptable ou en sel d'ammonium quaternaire de la pipéridine.

**10.** Compositions pharmaceutiques contenant comme principe actif au moins un composé selon l'une quelconque des revendications 1 à 7, seul ou en combinaison avec un ou plusieurs excipients ou véhicules inertes, non toxiques, pharmaceutiquement acceptables.

**11.** Compositions pharmaceutiques selon la revendication 10 contenant au moins un principe actif selon l'une quelconque des revendications 1 à 7 utiles comme antagonistes des récepteurs $NK_1$ dans le traitement de la douleur, de l'inflammation, des troubles gastro-intestinaux ou urinaires, de l'asthme, des allergies, de la migraine et des maladies du système nerveux central.

**Patentansprüche**

**1.** Verbindung der Formel (I):

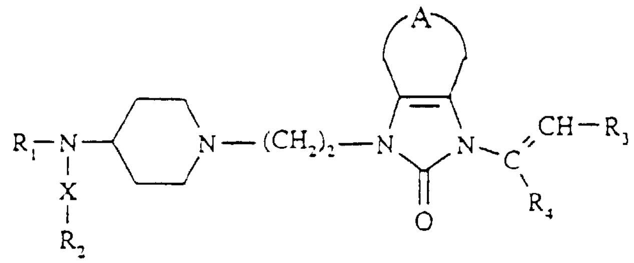

(I)

in der:

$R_1$ eine geradkettige oder verzweigte ($C_1$-$C_6$)-Alkylgruppe, eine Phenylgruppe, eine Naphthylgruppe, eine Pyridylgruppe oder eine Thienylgruppe, wobei jede Phenyl-, Naphthyl-, Pyridyl- oder Thienylgruppe gegebenenfalls durch ein oder mehrere Halogenatome oder Hydroxygruppen, geradkettige oder verzweigte ($C_1$-$C_6$)-Alkoxygruppen, geradkettige oder verzweigte ($C_1$-$C_6$)-Alkylgruppen, Trihalogenmethylgruppen oder 1-Hydroxy-2,2,2-trifluorethylgruppen substituiert ist.

$R_2$ ein Wasserstoffatom, eine geradkettige oder verzweigte ($C_1$-$C_6$)-Alkylgruppe (die gegebenenfalls durch eine oder mehrere geradkettige oder verzweigte ($C_1$-$C_6$)-Alkoxygruppen oder Phenylgruppen, Aminogruppen oder Phthalimidogruppen substituiert ist), eine Phenylgruppe (die gegebenenfalls durch ein oder mehrere Halogenatome oder eine oder mehrere geradkettige oder verzweigte ($C_1$-$C_6$)-Alkylgruppen, geradkettige oder verzweigte ($C_1$-$C_6$)-Alkoxygruppen. Hydroxygruppen oder Trihalogenmethylgruppen substituiert ist), eine ($C_3$-$C_7$) -Cycloalkylgruppe, eine Piperidinogruppe oder eine Aminogruppe (die gegebenenfalls durch eine oder mehrere geradkettige oder verzweigte ($C_1$-$C_6$)-Alkylgruppen substituiert ist),

X eine Gruppe CO oder $SO_2$,

$R_3$ ein Wasserstoffatom oder eine geradkettige oder verzweigte ($C_1$-$C_6$)-Alkylgruppe,

$R_4$ eine geradkettige oder verzweigte ($C_1$-$C_6$)-Alkylgruppe, eine Phenylgruppe (die gegebenenfalls durch ein odermehrere Halogenatome oder eine oder mehrere geradkettige oder verzweigte ($C_1$-$C_6$)-Alkylgruppen, geradkettige oder verzweigte ($C_1$-$C_6$)-Alkoxygruppen. Hydroxygruppen oder Trihalogenmethylgruppen substituiert ist) oder eine Trihalogenmethylgruppe,

oder $R_3$ und $R_4$ gemeinsam mit den sie tragenden Kohlenstoffatomen eine ($C_3$-$C_7$)-Cycloalkenylgruppe und

A mit den Kohlenstoffatomen, an die es gebunden ist, einen Phenyl-, Naphthyl- oder Pyridylring, wobei jeder Phenyl-, Naphthyl- oder Pyridylring gegebenenfalls durch ein oder mehrere Halogenatome, eine oder mehrere geradkettige oder verzweigte ($C_1$-$C_6$)-Alkylgruppen, geradkettige oder verzweigte ($C_1$-$C_6$)-Alkoxygruppen, Hydroxygruppen, Aminogruppen, Nitrogruppen oder Trihalogenmethylgruppen substituiert ist,

bedeuten,
deren Isomeren, deren entsprechende quartären Ammoniumsalze des Piperidins sowie deren Additionssalze mit einer pharmazeutisch annehmbaren Säure.

**2.** Verbindung der Formel (I) nach Anspruch 1, in der X eine CO-Gruppe bedeutet.

**3.** Verbindung der Formel (I) nach Anspruch 1, in der $R_3$ ein Wasserstoffatom bedeutet.

**4.** Verbindung der Formel (I) nach Anspruch 1, in der $R_4$ eine geradkettige oder verzweigte ($C_1$-$C_6$)-Alkylgruppe bedeutet.

**5.** Verbindung der Formel (I) nach Anspruch 1, in der A einen gegebenenfalls durch ein oder mehrere Halogenatome oder eine oder mehrere geradkettige oder verzweigte ($C_1$-$C_6$)-Alkylgruppen, geradkettige oder verzweigte ($C_1$-$C_6$)-Alkoxygruppen, Hydroxygruppen, Aminogruppen, Nitrogruppen oder Trihalogenmethylgruppen substituierten Phenylring bedeutet.

**6.** Verbindung der Formel (I) nach Anspruch 1, nämlich 1-{2-[4-(N-Propionyl-(3,4-dichlorphenyl)-amino)-piperidino]-ethyl}-3-isopropenyl-2(3H)-benzimidazolon.

**7.** Verbindung der Formel (I) nach Anspruch 1, nämlich 1-{2-[4-(N-Methoxyacetyl-(3,4-dichlorphenyl)-amino)-piperidino]-ethyl)-3-isopropenyl-2(3H)-benzimidazolon.

**8.** Verfahren zur Herstellung der Verbindungen der Formel (I) nach Anspruch 1, dadurch gekennzeichnet, daß man als Ausgangsprodukt ein Amin der Formel (II) verwendet:

$$R_1 - NH_2 \quad (II)$$

in der $R_1$ die bezüglich der Formel (I) angegebenen Bedeutungen besitzt,
welches man mit einem Piperidon der Formel (III) umsetzt:

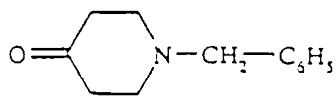

(III)

zur Bildung der Verbindung der Formel (IV):

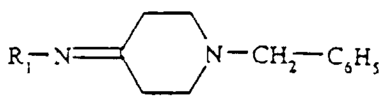

(IV)

in der $R_1$ die bezüglich der Formel (I) angegebenen Bedeutungen besitzt,
welche man einer Reduktion in Gegenwart eines Metallhydrids unterwirft zur Bildung der Verbindung der Formel (V):

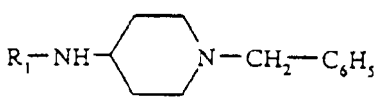

(V)

in der $R_1$ die bezüglich der Formel (I) angegebenen Bedeutungen besitzt,
welche man mit einem Anhydrid, Säurechlorid oder Phosgen (gefolgt von einer Reaktion mit einem sekundären Amin) unterwirft) zur Bildung der Verbindung der Formel (VI):

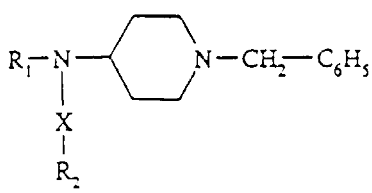

(VI)

in der $R_1$, $R_2$ und X die bezüglich der Formel (I) angegebenen Bedeutungen besitzen,
welche man einer Debenzylierung unterwirft durch katalytische Hydrierung. Wasserstoffübertragung (in Gegenwart von Ammoniumformiat) oder durch Dealkylierung (in Gegenwart von α-Chlorameisensäurechlorethylester)
zur Bildung der Verbindung der Formel (VII):

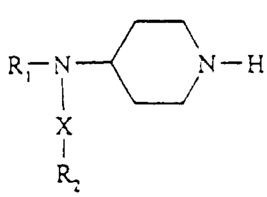

(VII)

in der $R_1$, $R_2$ und X die bezüglich der Formel (I) angegebenen Bedeutungen besitzt, welche man mit einer Verbindung der Formel (VIII) umsetzt:

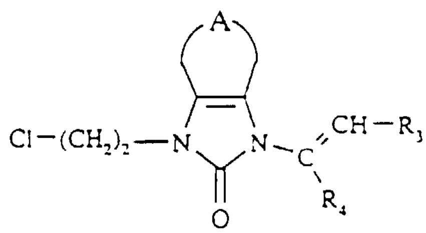

(VIII)

in der A, $R_3$ und $R_4$ die bezüglich der Formel (I) angegebenen Bedeutungen besitzen, zur Bildung der Verbindung der Formel (I),

welche Verbindung der Formel (I) man:

- gegebenenfalls mit Hilfe einer klassischen Reinigungsmethode reinigen kan,
- gegebenenfalls mit Hilfe einer klassischen Trennmethode in die Isomeren auftrenen kann und
- welche man gewünschtenfalls in ihre Additionssalze mit einer pharmazeutisch annehmbaren Säure oder das quaternäre Ammoniumsalz des Piperidins umwandeln kann.

**9.** Verfahren zur Herstellung der Verbindungen der Formel (I) nach Anspruch 1, dadurch gekennzeichnet, daß man als Ausgangsprodukt ein Piperidin der Formel (IX) verwendet:

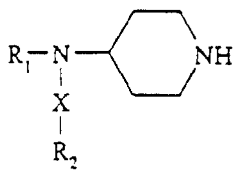

(IX)

in der $R_1$, $R_2$ und X die bezüglich der Formel (I) angegebenen Bedeutungen besitzen, welche man mit Epoxyethan umsetzt zur Bildung der Verbindung der Formel (X):

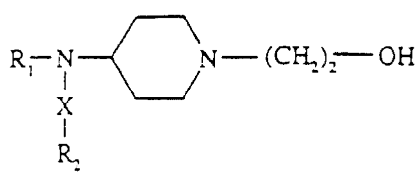

(X)

in der $R_1$, $R_2$ und X die bezüglich der Formel (I) angegebenen Bedeutungen besitzen, welche man der Einwirkung von Thionylchlorid unterzieht zur Bildung der Verbindung der Formel (XI):

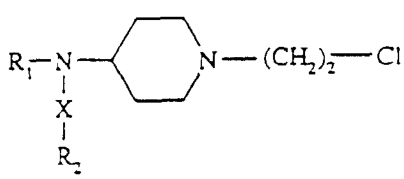

(XI)

in der $R_1$, $R_2$ und X die bezüglich der Formel (I) angegebenen Bedeutungen besitzen, welche man mit der Verbindung der Formel (XII) umsetzt:

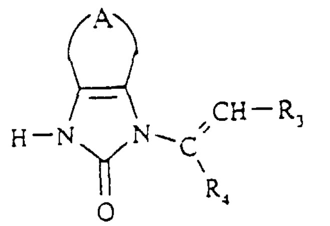

(XII)

in der A, $R_3$ und $R_4$ die bezüglich der Formel (I) angegebenen Bedeutungen besitzen, zur Bildung der Verbindung der Formel (I), welche Verbindung der Formel (I):

- gegebenenfalls mit Hilfe einer klassischen Reinigungsmethode gereinigt werden kann,
- gegebenenfalls mit Hilfe einer klassischen Trennmethode in die Isomeren aufgetrennt werden kann und
- gewünschtenfalls in ihre Additionssalze mit einer pharmazeutisch annehmbaren Säure oder ihr quartäres Ammoniumsalz des Piperidins umgewandelt werden kann.

**10.** Pharmazeutische Zubereitungen enthaltend als Wirkstoff mindestens eine Verbindung nach einem der Ansprüche 1 bis 7 allein oder in Kombination mit einem oder mehreren inerten, nichttoxischen, pharmazeutisch annehmbaren Trägermaterialien oder Bindemitteln.

**11.** Pharmazeutische Zubereitungen nach Anspruch 10 enthaltend mindestens einen Wirkstoff nach einem der Ansprüche 1 bis 7 geeignet als Antagonisten der $NK_1$-Rezeptoren bei der Behandlung von Schmerzen, Entzündungen, Magen-Darm-Störungen oder Harnwegstörungen, Asthma, Allergien, Migräne und Erkrankungen des Zentralnervensystems.

**Claims**

**1.** Compound of formula (I):

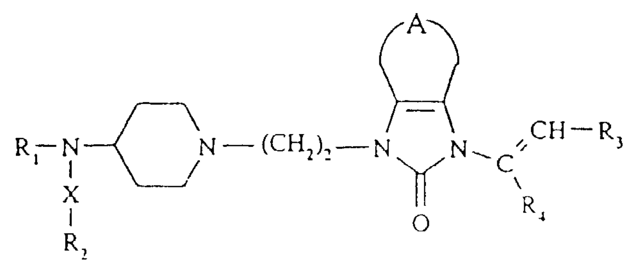

(I)

wherein :

$R_1$ represents a linear or branched ($C_1$-$C_6$)alkyl group or a phenyl, naphthyl, pyridyl or thienyl group, each phenyl, naphthyl, pyridyl or thienyl group optionally being substituted by one or more halogen atoms or hydroxy, linear or branched ($C_1$-$C_6$)-alkoxy, linear or branched ($C_1$-$C_6$)alkyl, trihalomethyl or 1-hydroxy-2,2,2-trifluoroethyl groups,

$R_2$ represents a hydrogen atom, a linear or branched ($C_1$-$C_6$)alkyl group (unsubstituted or substituted by one or more linear or branched ($C_1$-$C_6$)alkoxy or phenyl, amino or phthalimido groups), phenyl (unsubstituted or substituted by one or more halogen atoms or linear or branched ($C_1$-$C_6$)alkyl, linear or branched ($C_1$-$C_6$) alkoxy, hydroxy or trihalomethyl groups), ($C_3$-$C_7$)cycloalkyl, piperidino or amino (unsubstituted or substituted by one or two linear or branched ($C_1$-$C_6$)alkyl groups),

X represents a CO or $SO_2$ group,

$R_3$ represents a hydrogen atom or a linear or branched ($C_1$-$C_6$)alkyl group,

$R_4$ represents a linear or branched ($C_1$-$C_6$)alkyl group, a phenyl group (unsubstituted or substituted by one or more halogen atoms or linear or branched ($C_1$-$C_6$)alkyl, linear or branched ($C_1$-$C_6$)alkoxy, hydroxy or trihalomethyl groups) or a trihalomethyl group,

or $R_3$ and $R_4$ form together with the carbon atoms carrying them a ($C_3$-$C_7$)cycloalkenyl group,

A represents, with the carbon atoms to which it is attached, a phenyl, naphthyl or pyridyl ring, each phenyl, naphthyl or pyridyl ring optionally being substituted by one or more halogen atoms or linear or branched ($C_1$-$C_6$)alkyl, linear or branched ($C_1$-$C_6$)alkoxy, hydroxy, amino, nitro or trihalomethyl groups,

their isomers, the corresponding piperidine quaternary ammonium salts and also the addition salts thereof with a pharmaceutically acceptable acid.

**2.** Compound of formula (I) according to claim 1, wherein X represents a CO group.

**3.** Compound of formula (I) according to claim 1, wherein $R_3$ represents a hydrogen atom.

**4.** Compound of formula (I) according to claim 1, wherein $R_4$ represents a linear or branched ($C_1$-$C_6$)alkyl group.

**5.** Compound of formula (I) according to claim 1, wherein A represents a phenyl ring optionally substituted by one or more halogen atoms or linear or branched ($C_1$-$C_6$)-alkyl, linear or branched ($C_1$-$C_6$)alkoxy, hydroxy, amino, nitro or trihalomethyl groups.

**6.** Compound of formula (I) according to claim 1, which is 1-{2-[4-(N-propionyl-(3,4-dichlorophenyl)amino)piperidino] ethyl}-3-isopropenyl-2(3H)-benzimidazolone.

**7.** Compound of formula (I) according to claim 1, which is 1-{2-[4-(N-methoxyacetyl-(3,4-dichlorophenyl)amino)piperidino]ethyl}-3-isopropenyl-2(3H)-benzimidazolone.

**8.** Process for the preparation of compounds of formula (I) according to claim 1, characterised in that there is used as starting material an amine of formula (II) :

$$R_1\text{-}NH_2 \qquad (II)$$

wherein $R_1$ is as defined for formula (I),
which is reacted with a piperidone of formula (III):

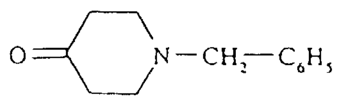

(III)

to yield a compound of formula (IV)

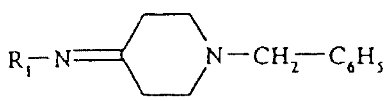

(IV)

wherein $R_1$ is as defined for formula (I),
which is subjected to reduction in the presence of a metal hydride,
to yield a compound of formula (V):

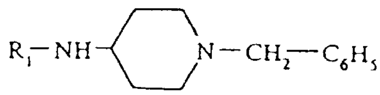

(V)

wherein $R_1$ is as defined for formula (I),

which is reacted with an anhydride, an acid chloride or phosgene (followed by a reaction with a secondary amine),

to yield a compound of formula (VI):

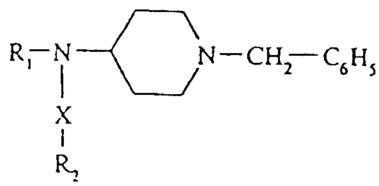

(VI)

wherein $R_1$, $R_2$ and X are as defined for formula (I),

which is subjected to debenzylation by catalytic hydrogenation, hydrogen transfer (in the presence of ammonium formate) or by dealkylation (in the presence of α-chloroethyl chloroformate),

to yield a compound of formula (VII)

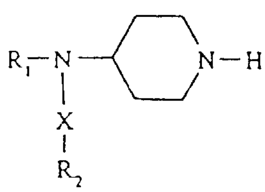

(VII)

wherein $R_1$, $R_2$ and X are as defined for formula (I),

which is reacted with a compound of formula (VIII):

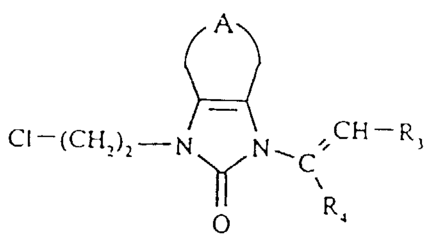

(VIII)

wherein A, $R_3$ and $R_4$ are as defined for formula (I),

to yield a compound of formula (I),

which compound of formula (I):

- may, if necessary, be purified according to a conventional purification technique,
- is, where appropriate, separated into its isomers according to a conventional separation technique,
- is, if desired, converted into an addition salt with a pharmaceutically acceptable acid or into a piperidine quaternary ammonium salt.

**9.** Process for the preparation of compounds of formula (I) according to claim 1, characterised in that there is used as starting material a piperidine of formula (IX):

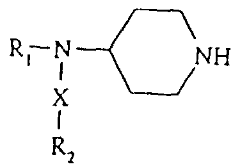

(IX)

wherein $R_1$, $R_2$ and X are as defined for formula (I) which is reacted with epoxyethane to yield a compound of formula (X) :

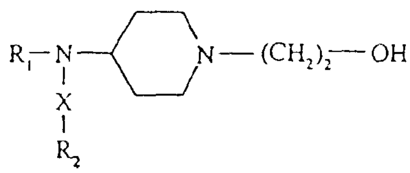

(X)

wherein $R_1$, $R_2$ and X are as defined for formula (I), which is subjected to the action of thionyl chloride to yield a compound of formula (XI):

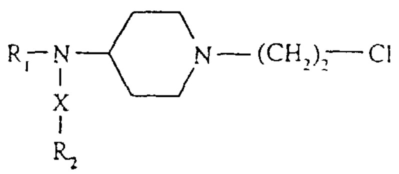

(XI)

wherein $R_1$, $R_2$ and X are as defined for formula (I), which is reacted with a compound of formula (XII):

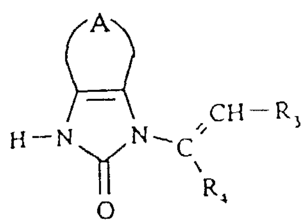

(XII)

wherein A, $R_3$ and $R_4$ are as defined for formula (I), to yield a compound of formula (I),

which compound of formula (I):

- may, if necessary, be purified according to a conventional purification technique,
- is, where appropriate, separated into its isomers according to a conventional separation technique,
- is, if desired, converted into an addition salt with a pharmaceutically acceptable acid or into a piperidine quaternary ammonium salt.

**10.** Pharmaceutical compositions comprising as active ingredient at least one compound according to any one of claims 1 to 7 alone or in combination with one or more pharmaceutically acceptable, inert, non-toxic excipients or carriers.

**11.** Pharmaceutical compositions according to claim 10 containing at least one active ingredient according to any one of claims 1 to 7, for use as $NK_1$ receptor antagonists in the treatment of pain, inflammation, gastro-intestinal or urinary disorders, asthma, allergies, migraine and central nervous system disorders.